# EUROPEAN PATENT SPECIFICATION

(11) **EP 0 615 486 B1**
(45) Date of publication and mention of the grant of the patent: **30.09.1998**
(21) Application number: 92923913.5
(22) Date of filing: 25.11.1992
(51) Int. Cl.: B29C 65/02, C08J 5/12, A61L 9/12, B65D 77/20, B65B 7/28

(54) **IMPROVEMENTS IN OR RELATING TO WELDING**
VERBESSERUNGEN IN VERBINDUNG MIT SCHWEISSEN
AMELIORATIONS RELATIVES A DES SOUDURES

(30) Priority: 27.11.1991 GB 9125253
(43) Date of publication of application: 21.09.1994
(73) Proprietor: RECKITT & COLMAN PRODUCTS LIMITED, London W4 2RW (GB)
(72) Inventor: WALLWORK, Stephen, Fred, Bolton BL1 5BD (GB)
(74) Representative: Sanderson, Nigel Paul
(86) International application number: GB9202178
(87) International publication number: WO9310959

(56) References cited:
- EP-A- 93 262
- EP-A- 260 896
- WO-A-84/02654
- WO-A-90/11177
- CH-A- 426 225
- FR-A- 2 019 461
- FR-A- 2 202 783
- FR-A- 2 204 493
- FR-A- 2 637 265
- GB-A- 2 053 794
- US-A- 3 533 548
- US-A- 4 257 530
- US-A- 4 991 375

## Description

This invention relates to welding and, more specifically, to the fabrication of articles by welding processes.

It is well known in the art to fabricate an article by welding together two or are elements, particularly when said elements are of the same or similar materials, but problems do exist when the elements are of different materials and wherein the temperature and/or pressure required to bring one of the said elements into a condition for welding may adversely affect the other element. This problem is compounded when three or more elements of different materials are to be welded together by a single welding process.

By way of example, problems do arise in the welding of paper to the higher melting temperature thermoplastics materials and wherein the temperature required for welding the thermoplastics material approaches the combustion temperature for the paper. In this specification the term " high melting temperature thermoplastics materials" is intended to mean thermoplastics materials having a melting temperature of at least 130 degrees C.

The problems of heat sealing or welding an olefinic polymer to a surface consisting of a polar synthetic or cellulose material are recognised in, for example, British Patent Publication No 2053794-A and in that publication it is claimed that the problem can be solved by subjecting the olefinic plastics to a corona treatment before the welding of the polymer to the polar synthetic or cellulose material is effected.

The applicant has discovered that a thermoplastics material can be welded to a cellulose material without first subjecting the plastics material to a corona treatment and, accordingly, all welding processes as require a corona treatment before the welding is affected are disclaimed herefrom.

It is well known in the art to fabricate devices for dispensing vapours of volatile liquids and wherein the liquid is retained in a container open at one side and the said open side is closed by a vapour-permeable, liquid-impermeable membrane. One form of vapour-permeable, liquid-impermeable vapour membrane which offers particular advantages in this art comprises a sheet of paper coated or impregnated, hereinafter called coated, with a material to render the laminate membrane liquid-impermeable but vapour-permeable when the coated surface of the laminate is exposed to atmosphere. One such device is disclosed in British Patent Publication No. 2194889.

The container for such a device must essentially be inert with respect to the volatile liquid to be contained.

Polypropylene is one material inert with respect to many volatile liquids to be dispensed as vapours for household purposes, such as perfumes and the like; air fresheners; liquid insecticides; liquid insect repellents; liquid deodorants; liquid corrosion inhibitors; germicidal agents; and medicants such as respiration-aiding liquids and, being readily mouldable and relatively inexpensive, is widely used for household volatile liquid dispensing devices.

However, in view of the difficulties identified above in the welding of paper to thermoplastics materials, such as polypropylene, and the delicate nature of the liquid-impermeable, vapour-permeable coating of the laminate, it has always been considered that the laminate could not withstand the temperatures and pressures necessary for welding to a high melting temperature material such as a polypropylene material.

For this reason all such volatile liquid dispensing devices including a vapour-permeable, liquid-impermeable laminate proposed to date for household use have used a mechanical device, to circumferentially trap the laminate over the open end of the reservoir, or an adhesive to secure the laminate across the open end of the liquid reservoir.

Mechanical devices for trapping the laminates are unreliable, particularly when the laminates are relatively delicate, and can add substantially to the costs of the dispensing devices. Whilst adhesives are more reliable, and cheaper, than mechanical devices, the use of such adhesives in an industrial process is messy, the process must be carefully controlled to avoid leak paths for the volatile liquid and, because of the wide range of compositions of volatile liquids to be dispensed by such devices, it is necessary to select, for each volatile material, an adhesive resistant to attack by that volatile liquid.

The present invention seeks to provide a method for welding an element comprising paper to a polypropylene or the like relatively high melting temperature thermoplastics material.

According to the present invention there is provided a method for welding an element, comprising a paper sheet having a coating of a vapour-permeable liquid-impermeable material, to a thermoplastics material so that the coating is remote from the thermoplastic material and wherein the thermoplastics material has not been subjected to a Corona treatment, which method comprises the steps of applying the element to a surface of the thermoplastics material, applying a pressure in the range of 150 kPa to 2000 kPa to the interface of the element with the thermoplastics material, and subjecting the said pressure-loaded plastics material and element to an elevated temperature in the range of from 140 degrees C. to 300 degrees C.

Preferably the method is characterised by the steps of subjecting the element and the thermoplastics material to a temperature of from 160 degrees C. to 240 degrees C. and, more preferably, to a temperature of from 175 degrees C. to 220 degrees C.

Preferably the method is characterised by the steps of subjecting the interface between the element and the thermoplastics material to a pressure in the range of 150 kPa to 500 kPa and more preferably to a pressure in the range of 200 kPa to 450 kPa.

Preferably the method is characterised by the steps of maintaining the interface between the element and the thermoplastics material at said elevated temperature and pressure for a period of from 0.5 seconds to 20 seconds. more preferably for a period of from 5 seconds to 15 seconds.

Preferably the method includes the steps of selecting the thermoplastics material to have a softening point below 280 degrees C., more preferably below 220 degrees C., and a melt-flow index of from 4 to 100.

In a preferred embodiment the method includes the step of coating one major surface of the paper sheet with a layer of a clay material and coating the major face of the clay layer remote from the paper with the coating material.

Preferably the vapour-permeable, liquid-impermeable material is an organopolysiloxane material, and most preferably a silicone elastomer.

In one embodiment the method includes the step of welding the said element to the thermoplastics material in a single welding operation.

In another embodiment the method includes the step of welding the said element to the thermoplastics material by a multi-stage welding operation.

In a preferred embodiment the method includes the step of moulding the thermoplastics material to form a container before welding the element thereto.

Preferably the method includes the steps of moulding the container to be open at one end and welding the said element to a surface of the container surrounding the open end of the said container.

Preferably the method includes the step of moulding the container to define a flange surrounding the open end of the container and welding said element to said flange.

Preferably the method includes the step of charging the said container with a volatile material.

In a preferred embodiment the method includes the step of applying a vapour-impermeable, liquid-impermeable layer of material over said element.

The invention will now be described further by way of example with reference to the accompanying drawings in which,
- Fig. 1: shows an axial, exploded cross-sectional view of one volatile liquid dispensing device located in a welding apparatus for welding in accordance with the invention,
- Fig. 2: shows a view of the device illustrated in Fig. 1 in the direction of the arrow A after the welding of a laminate to close the open end of the device, and
- Fig. 3: shows a detail cross-sectional view through one edge region of the device shown in Fig. 2 with a liquid-impermeable, vapour-impermeable barrier element applied thereto.

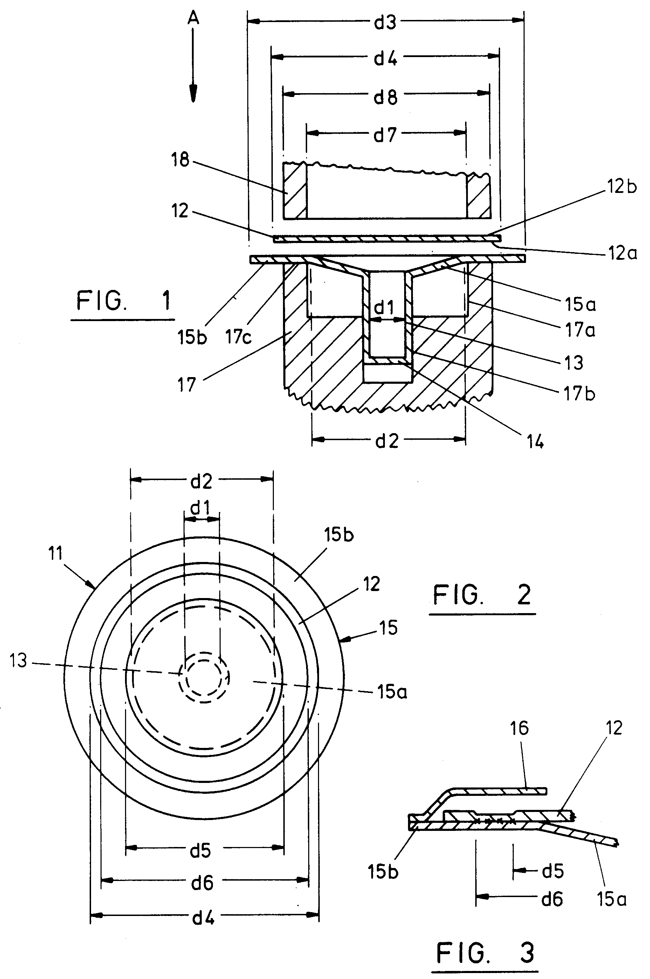

The device shown in Figs. 1, 2 and 3 of the drawings generally comprises a container 11, defining a reservoir open at one end, and an element comprising a vapour-permeable, liquid-impermeable laminate 12.

The container 11 comprises a body of revolution defined by a cylindrical wall 13, closed at one end by a rigid end wall 14, with a flange 15 extending generally radially from that end of the cylindrical wall 13 remote from the end wall 14.

As will be most readily seen from Figs. 1 and 2 the flange 15 comprises an inner wall region 15a, between diameters d1 and d2, which slopes upwardly and outwardly from the upper regions of the cylindrical wall 13 (as viewed in Fig. 1), so as to be inclined to the horizontal when the axis of the cylindrical wall 13 is vertical. The flange 15 further includes an outer annular region 15b, between diameters d2 and d3, lying in a plane at right angles to the axis of the cylindrical wall 13.

The cross-sectional area of the flange 15b on that side of flange 15 to be contacted by the element 12 is conveniently greater than twice the cross-sectional area of the reservoir opening d2 at the said flange side.

The container 11 is conveniently moulded from a polypropylene material, and most preferably said container 11 comprises a see-through polypropylene material, through which the level of volatile liquid within the container 11 can be readily seen.

The vapour-permeable, liquid-impermeable laminate 12 is of disc-like form and presents parallel major faces 12a and 12b. Said element 12 is made from a porous sheet of paper treated on its surface 12b, with a material, conveniently a substantially elastomeric crosslinked organopolysiloxane (silicone) material, which renders the laminate 12 impermeable to the liquid but vapour-permeable to permit the passage of vapours therethrough.

The paper must be robust and mechanically strong and must also have a high wet strength, as the evaporation of volatile liquid at a higher rate than its replacement by air can cause cavitation in the element 12.

Such a composite laminate is fully described in the above identified British Patent Publication No. GB 2194889.

In another form the laminate 12 conveniently comprises a layer of clay between the said sheet of paper and said treatment material.

The vapour-permeable, liquid-impermeable element 12 has an outer diameter d4 less than the diameter d3 of the flange 15b, whereupon the element 12 can be welded to the flange 15b around an annulus between diameter d5 and d6, the smaller diameter d5 being greater than the diameter d2 and the larger diameter d6 being less than the diameter d3. The diameter of d5 is calculated to define an area of the surface 12a of the element 12 exposed to the open end of the reservoir defined by the container 11 and which has been pre-determined to obtain the desired rate of release of the vapours for the volatile liquid contained in the container 11.

The reservoir container 11 may be charged with volatile material before the laminate 12 is applied thereto but in a preferred alternative method the laminate 12 is welded to the flange 15b, and the reservoir is charged through an aperture (not shown) in the end wall 14 and which aperture is then closed with a non-peelable foil, for example an aluminium foil laminated with polypropylene, to prevent the loss of volatile liquid and volatile liquid vapours through said aperture.

With the laminate 12 welded to the container 11 a dish-shaped, liquid-impermeable vapour-impermeable barrier element 16, conveniently made from a metallic foil inert with respect to the volatile liquid in the container or coated with a material to render the element 16 inert with respect to said volatile liquid, has its peripheral edge regions welded or otherwise bonded to the edge regions of the flange 15b outwardly of the laminate 12. The barrier element 16 serves to prevent the loss of volatile liquid from the reservoir whilst the device is in storage and is peeled from the device when the device is to be brought into use.

The laminate 12 is welded to the flange 15b by locating the casing 11 in a cylindrical welding die 17, which presents an annular recess 17a with an axial bore region 17b for receiving the cylindrical wall 13 of the container 11 therein. The die 17 thus defines an annular wall 17c which supports the underside of the flange 15b when the elements are in the position shown in Fig. 1. The inner diameter d7 of the annular wall 17c defined by the recess 17a is equal to the diameter d5 of the desired inner diameter of the weld annulus and the outer diameter d8 of the annular wall 17c is equal to the diameter d6 of the desired outer diameter of the die 17.

The welding apparatus further includes a second annular die 18, above and axially aligned with the die 17 and arranged for reciprocation, by means not shown in the drawings, towards and away from the annular die 17. The inner diameter of the die 18 is equal to the inner diameter d7 of the die 17 and the outer diameter of the die 18 is equal to the outer diameter d8 of the die 17.

With the dies 17 and 18 separated a casing 11 is located in the die 17 with its cylindrical wall 13 snugly received in the bore 17b a laminate 12 is placed on the flange 15b with its surface 12a in contact with flange 15b and the die 18 is then lowered to make pressure contact with the laminate 12.

Thereafter, and in accordance with the invention, the pressure exerted by die 18 on the laminate 12 is adjusted to produce a pressure on the laminate 12 in the region of 150 kPa to 2000 kPa and the die 17 and/or the die 18 is heated to heat that part of flange 15b and that part of laminate 12 between the dies 17 and 18, to a temperature in the range of 140 degrees C to 300 degrees C.

The pressure between the dies 17 and 18 is maintained for a period of at least 0.5 seconds and, thereafter, the die 18 is elevated to allow the container 11 with laminate 12 welded thereto to be removed from the die 17.

For one set of trials the die 18 included a thermostatically controlled heater, which allowed the temperature of the die 18 to be adjustable whereupon the die 18 could apply a desired temperature to the parts to be welded.

Trials were carried out on four different materials, as follows;
(1) High density polyethylene (HDP) (extrusion blow moulding grade).
(2) High impact polystyrene (HIP) having a melt flow index of 4.
(3) High impact polystyrene (HIP) having a melt flow index of 18, and
(4) A polypropylene material.

The thickness of the thermoplastic material was, for material (1) about 3 mm, for materials (2) and (3) about 1.25 mm and for material (4) about 1.5 mm for each of the trials and the laminate material for each trial had a thickness of 160 microns and a weight of 137 g/m².

For each trial the temperature of the die was allowed to stabilise at the desired temperature before the dies 17 and 18 were closed to weld the laminate to the thermoplastics.

To test the weld the welded laminate/polymer samples were pulled open at the weld interface and a weld was deemed successful if a significant amount of paper residue was left as a continuous bond around the weld line (lower limit)

The upper limit of suitability with respect to temperature was determined by the extent to which the paper degraded and changed colour.

A number of experiments were carried out with the four materials listed, varying the temperature, pressure and time for which temperature and pressure were applied. The results are shown in Table 1.

**TABLE 1**

| MATERIAL | SEAL LIMITS | TIME (SECS) | TEMP (°C) | PRESSURE kPa |
|---|---|---|---|---|
| (1) | LOWER | 15 | 200 | 400 |
| | | 5 | 220 | 400 |
| | UPPER | 20 | 200 | 250 |
| | | 5 | 220 | 250 |
| (2) | LOWER | 10 | 180 | 400 |
| | UPPER | 10 | 200 | 250 |
| (3) | LOWER | 5 | 180 | 400 |
| | UPPER | 10 | 200 | 250 |
| (4) | LOWER | 10 | 185 | 460 |
| | UPPER | 5 | 220 | 460 |

It should be noted that when the laminate 12 is in compression between the die 18 and the flange 15b a large number of the paper fibres in laminate 12 are brought into welding contact with the flange 15b and, when the welding process has been completed, the thickness of the laminate 12 between the diameters d5 and d6 will be permanently reduced relative to the thickness of the remaining parts of the laminate 12. Some flow of the flange 15b material into the laminate 12 will have taken place but flow paths for vapour will still exist through the laminate 12 parallel to the plane of the element 12.

As stated hereinbefore, the container 11 can be charged with volatile material before the laminate 12 is welded thereto but most preferably the container 11 is charged after the laminate 12 is welded thereto, to avoid heating of the volatile liquid by the welding process and consequential loss of volatile liquid prior to the bonding of the barrier element 16 to the assembly.

To prepare the dispensing device for use the barrier layer 16 is first peeled off, to expose the surface 12b of the laminate 12 to the surrounding atmosphere, and the device inverted from the position shown in Fig. 1 and is supported on stand means so that air can flow readily over the surface 12b of the laminate 12. In this position the volatile liquid in the container 11 rests on the surface 12a of the laminate 12 within the diameter d5 and the volatile liquid vaporises through the liquid-impermeable, vapour-permeable coating on the surface 12b to the surrounding atmosphere.

## Claims

1. A method for welding an element, comprising a paper sheet having a coating of a vapour-permeable liquid-impermeable material, to a thermoplastics material so that the coating is remote from the thermoplastic material and wherein the thermoplastics material has not been subjected to a Corona treatment, which method comprises the steps of applying the element to a surface of the thermoplastics material, applying a pressure in the range of 150 kPa to 2000 kPa to the interface of the element with the thermoplastics material, and subjecting the said pressure-loaded plastics material and element to an elevated temperature in the range of from 140 degrees C. to 300 degrees C.

2. A method as claimed in claim 1, wherein the interface between the element and the thermoplastics material is subjected to a temperature of from 160 degrees C. to 240 degrees C.

3. A method as claimed in claim 2, wherein the interface between the element and the thermoplastics material is subjected to a temperature of from 175 degrees C. to 220 degrees C.

4. A method as claimed in claim 1, 2 or 3, wherein a pressure of from 150 kPa to 500 kPa is applied to the interface.

5. A method as claimed in claim 4, wherein a pressure of from 200 kPa to 450 kPa is applied to the interface.

6. A method as claimed in any one of claims 1, 2, 3, 4 or 5, wherein the interface between the element and the thermoplastics material is maintained at said elevated temperature and pressure for a period of from 0.5 seconds to 20 seconds.

7. A method as claimed in claim 6, wherein the interface between the element and the thermoplastics material is maintained at said elevated temperature and pressure for a period of from 5 seconds to 15 seconds.

8. A method as claimed in any one of the preceding claims, wherein the thermoplastics material has a softening point below 280 degrees C. and a melt-flow index of from 4 to 100.

9. A method as claimed in claim 8, wherein the thermoplastics material has a softening point below 220 degrees C. and a melt-flow index of from 4 to 100.

10. A method as claimed in any one of claims 1 to 9 comprising the step of coating one major surface of the paper sheet with a layer of a clay material and coating the major face of the layer of clay material remote from the paper sheet with said vapour-permeable liquid-impermeable material.

11. A method as claimed in any preceding claim, wherein said vapour-permeable, liquid-impermeable material comprises an organopolysiloxane material.

12. A method as claimed in claim 11, wherein said organopolysiloxane material comprises a silicone elastomer.

13. A method as claimed in any one of the preceding claims, wherein said element is welded to the thermoplastics material in a single welding operation.

14. A method as claimed in any one of claims 1 to 12, wherein said element is welded to the thermoplastics material by a multi-stage welding operation.

15. A method according to any one of the preceding claims, including the step of moulding the thermoplastics material to form a container before welding the element thereto.

16. A method as claimed in claim 15, wherein the container is moulded to be open at one end and said element is welded to a surface of the container surrounding the open end.

17. A method as claimed in claim 16, wherein the container is moulded so as to define a flange surrounding the open end and said element is welded to said flange.

18. A method as claimed in claim 15, 16 or 17 and comprising the step of charging said container with a volatile material.

19. A method as claimed in any preceding claim and comprising the step of applying a vapour-impermeable, liquid impermeable layer material to said element.

## Patentansprüche

1. Verfahren zum Schweißen eines Teils umfassend eine Papierlage mit einer Beschichtung aus einem dampfdurchlässigen, flüssigkeitsundurchlässigen Material auf ein thermoplastisches Material, so dass die Beschichtung entfernt dem thermoplastischen Material zum Liegen kommt wobei das thermoplastische Material nicht einer Corona-Behandlung unterworfen wird, umfassend die Schritte:
Anbringen des Teils auf eine Oberfläche des thermoplastischen Materials,
Aufringen eines Drucks im Bereich von 150 bis 2000 kPa auf die Grenzschicht des Teils mit dem thermoplastischen Material und Unterwerfen des druckbelasteten Kunststoffmaterials und Teils einer erhöhten Temperatur im Bereich von 140°C bis 300°C.

2. Verfahren nach Anspruch 1, wobei die Grenzschicht zwischen dem Teil und dem thermoplastischen Material einer Temperatur von 160°C bis 240°C unterworfen wird.

3. Verfahren nach Anspruch 2, wobei die Grenzschicht zwischen dem Teil und dem thermoplastischen Material einer Temperatur von 175°C bis 220°C unterworfen wird.

4. Verfahren nach Anspruch 1, 2 oder 3, wobei der auf die Grenzschicht aufgebrachte Druck 150 bis 500 kPa beträgt.

5. Verfahren nach Anspruch 4, wobei der auf die Grenzschicht aufgebrachte Druck 200 bis 450 kPa beträgt.

6. Verfahren nach einem der Ansprüche 1, 2, 3, 4 oder 5, wobei die Grenzschicht zwischen dem Teil und dem thermoplastischen Material für 0,5 bis 20 Sekunden auf einer erhöhten Temperatur und unter einem Druck gehalten wird.

7. Verfahren nach Anspruch 6, wobei die Grenzschicht zwischen dem Teil und dem thermoplastischen Material für 5 bis 15 Sekunden auf einer erhöhten Temperatur und unter Druck gehalten wird.

8. Verfahren nach irgendeinem vorhergehenden Anspruch, wobei das thermoplastische Material einen Erweichungspunkt unter 280°C und eine Schmelzflusszahl von 4 bis 100 hat.

9. Verfahren nach Anspruch 8, wobei das thermoplastische Material einen Erweichungspunkt unter 220°C hat und eine Schmelzflusszahl von 4 bis 100.

10. Verfahren nach irgendeinem Anspruch 1 bis 9, umfassend den Schritt: Beschichten einer größeren Fläche der Papierlage mit einer Schicht aus Tonmaterial und Beschichten einer größeren Fläche der Lage aus Tonmaterial entfernt von der Papierlage mit dem dampfdurchlässigen, flüssigkeitsundurchlässigen Material.

11. Verfahren nach irgendeinem vorhergehenden Anspruch, wobei das dampfdurchlässige, flüssigkeitsundurchlässige Material ein Organopolysiloxan-Material umfasst.

12. Verfahren nach Anspruch 11, wobei das Organopolysiloxan-Material ein Silicon-Elastomer umfasst.

13. Verfahren nach irgendeinem vorhergehenden Anspruch, wobei das Teil in einem einzelnen Schweißvorgang an das thermoplastische Material geschweißt wird.

14. Verfahren nach irgendeinem Anspruch 1 bis 12, wobei das Teil in einem mehrstufigem Schweißvorgang an das thermoplastische Material geschweißt wird.

15. Verfahren nach irgendeinem vorhergehenden Anspruch, umfassend den Schritt: Formen des thermoplastischen Materials zu einem Behälter, bevor das Teil daran geschweißt wird.

16. Verfahren nach Anspruch 15, wobei der Behälter so geformt wird, dass er an einem Ende offen ist und das Element an die Oberfläche des Behälters, die das offene Ende umgibt, geschweißt wird.

17. Verfahren nach Anspruch 16, wobei der Behälter so geformt wird, dass er einen das offene Ende umgebenden Flansch definiert und das Teil an den Flansch geschweißt wird.

18. Verfahren nach Anspruch 15, 16 oder 17, umfassend den Schritt: Beladen des Behälters mit einem flüchtigen Material.

19. Verfahren nach irgendeinem vorhergehenden Anspruch umfassend den Schritt: Aufbringen einer dampfundurchlässigen, flüssigkeits-undurchlässigen Materiallage auf das Teil.

## Revendications

1. Procédé pour souder un élément, comportant une feuille de papier ayant un revêtement d'une matière perméable aux vapeurs et imperméable aux liquides, à une matière thermoplastique, de manière que le revêtement soit éloigné de la matière thermoplastique, et dans lequel la matière thermoplastique n'a pas été soumise à un traitement par effet corona, lequel procédé comprend les étapes d'application de l'élément sur une surface de la matière thermoplastique, d'application d'une pression dans la plage de 150 kPa à 2000 kPa à l'interface de l'élément avec la matière plastique, et de soumission de ladite matière plastique et de l'élément, soumis à la pression, à une température élevée dans la plage de 140°C à 300°C.

2. Procédé selon la revendication 1, dans lequel l'interface entre l'élément et la matière thermoplastique est soumise à une température de 160°C à 240°C.

3. Procédé selon la revendication 2, dans lequel l'interface entre l'élément et la matière thermoplastique est soumise à une température de 175°C à 220°C.

4. Procédé selon la revendication 1, 2 ou 3, dans lequel une pression de 150 kPa à 500 kPa est appliquée à l'interface.

5. Procédé selon la revendication 4, dans lequel une pression de 200 kPa à 450 kPa est appliquée à l'interface.

6. Procédé selon l'une quelconque des revendications 1, 2, 3, 4 ou 5, dans lequel l'interface entre l'élément et la matière thermoplastique est maintenue à ladite température élevée et à ladite pression pendant une période allant de 0,5 seconde à 20 secondes.

7. Procédé selon la revendication 6, dans lequel l'interface entre l'élément et la matière thermoplastique est maintenue à une température élevée et à ladite pression pendant une période allant de 5 secondes à 15 secondes.

8. Procédé selon l'une quelconque des revendications précédentes, dans lequel la matière thermoplastique présente un point de ramollissement inférieur à 280°C et un indice de fluidité à l'état fondu de 4 à 100.

9. Procédé selon la revendication 8, dans lequel la matière thermoplastique présente un point de ramollissement inférieur à 220°C et un indice de fluidité à l'état fondu de 4 à 100.

10. Procédé selon l'une quelconque des revendications 1 à 9, comprenant l'étape de revêtement d'une surface principale de la feuille de papier avec une couche de matière du type argile et de revêtement de la surface principale de la couche de matière du type argile, éloignée de la feuille de papier, avec ladite matière perméable aux vapeurs et imperméable aux liquides.

11. Procédé selon l'une quelconque des revendications précédentes, dans lequel ladite matière perméable aux vapeurs et imperméable aux liquides comprend une matière du type organopolysiloxane.

12. Procédé selon la revendication 11, dans lequel ladite matière du type organopolysiloxane comprend un élastomère de silicone.

13. Procédé selon l'une quelconque des revendications précédentes, dans lequel ledit élément est soudé à la matière thermoplastique en une seule opération de soudage.

14. Procédé selon l'une quelconque des revendications 1 à 12, dans lequel ledit élément est soudé à la matière thermoplastique par une opération de soudage à étapes multiples.

15. Procédé selon l'une quelconque des revendications précédentes, comprenant l'étape de moulage de la matière thermoplastique pour former un récipient avant le soudage de l'élément à cette matière.

16. Procédé selon la revendication 15, dans lequel le récipient est moulé de façon à être ouvert à une extrémité et ledit élément est soudé à une surface du récipient entourant l'extrémité ouverte.

17. Procédé selon la revendication 16, dans lequel le récipient est moulé de façon à définir un rebord entourant l'extrémité ouverte et ledit élément est soudé audit rebord.

18. Procédé selon la revendication 15, 16 ou 17 et comprenant l'étape de chargement dudit récipient d'une matière volatile.

19. Procédé selon l'une quelconque des revendications précédentes et comprenant l'étape d'application d'une matière formant une couche imperméable aux vapeurs et imperméable aux liquides audit élément.
